(19) Europäisches Patentamt
European Patent Office
Office européen des brevets

(11) Publication number: 0 442 424 B1

(12) EUROPEAN PATENT SPECIFICATION

(45) Date of publication of patent specification: **21.12.94** (51) Int. Cl.5: **A61K 31/505**, A61K 31/445

(21) Application number: **91101889.3**

(22) Date of filing: **11.02.91**

# (54) Treatment of sleep apneas with azapirone derivatives.

(30) Priority: **12.02.90 US 478820**

(43) Date of publication of application:
**21.08.91 Bulletin 91/34**

(45) Publication of the grant of the patent:
**21.12.94 Bulletin 94/51**

(84) Designated Contracting States:
**AT BE CH DE DK ES FR GB GR IT LI LU NL SE**

(56) References cited:
**US-A- 4 612 312**

**FASEB J. , vol. 2, no. 5, 1988, page A1507, abstract no. 7030; D.M. RAPOPORT etal.: "Comparison of the effects of buspirone and diazepam on control ofbreathing"**

**AM. REV. RESPIR. DIS., vol. 139, no. 4, April 1989, page A625; D.M. RAPOPORT etal.: "Bispirone - A new respiratory stimulant"**

**J. MED. CHEM., vol. 31, no. 7, 1988, pages 1382-1392; M. ABOU-GHARBIA et al.:"Polycyclic aryl- and heteroaryl-piperazinyl imides as 5-HT1A receptor ligands and potential anxiolytic agents:Synthesis and structure-activity relationshipstudies"**

**NEUROPSYCHOPHARMACOLOGY, vol. 3, no. 2, April 1990, pages 129-136, Elsevier Science Publishing Co., Inc.,New York, US; E. EDWARDS et al.: "5-HT1A and5HT1B agonists play a differential role on the respiratory frequency in rats"**

(73) Proprietor: **Bristol-Myers Squibb Company**
**345 Park Avenue**
**New York, N.Y. 10154 (US)**

(72) Inventor: **Schwimmer, Jeffrey L.**
**31 Egbert Avenue**
**Morristown, New Jersey 07960 (US)**

(74) Representative: **Kinzebach, Werner, Dr. et al**
**Patentanwälte**
**Reitstötter, Kinzebach und Partner**
**Postfach 86 06 49**
**D-81633 München (DE)**

**EP 0 442 424 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention is concerned with a drug bioaffecting body-treating process that employs members of the general "azapirone" structural class of psychotropic agents. The archetypical compound of the azapirone class of agents has the following structural formula

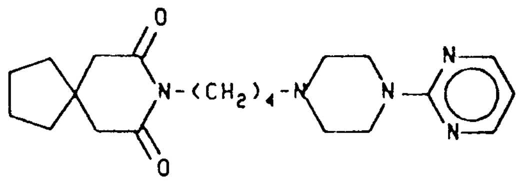

and is known as buspirone. The hydrochloride salt has been referred to in the prior art as MJ 9022-1 and as buspirone hydrochloride. Other acid addition salts thereof are named by combining "buspirone" with the appropriate word to define the acid from which it is prepared as in "buspirone hydrochloride". The latter is the United States Adopted Name (USAN); refer to J. American Med. Assoc. 225, 520 (1973).

The synthesis of the compound and the disclosure of its psychotropic properties are described in the following patents and publications.

1. Y.H. Wu, et al., J. Med. Chem., 15,477 (1972).
2. Y.H. Wu, et al., U.S. Pat. No. 3,717,634 which issued February 20, 1973.
3. L.E. Allen et al., Arzneium. Forsch., 24, No. 6, 917-922 (1974).
4. G.L. Sathananthan, et al., Current Therapeutic Research, 18/5, 701-705 (1975).
5. Y.H. Wu, et al., U.S. Pat. No. 3,976,776, issued August 24, 1976.

The following patent references disclose and claim additional uses that relate to buspirone's pharmacological effects on the central nervous system.

6. The use of buspirone hydrochloride as a novel antianxiety agent for the treatment of neurotic patients is described in G.P. Casten, et al., U.S. Patent No. 4,182,763, issued January 9, 1980.
7. Allen, et al., disclose the use of buspirone in treating extrapyramidal motor disorders in U.S. 4,438,119, issued March 20, 1984.
8. Buspirone's use in sexual dysfunction was described by Othmer, et al., in U.S. 4,640,921, issued February 3, 1987.
9. Kurtz, et al., in U.S. 4,634,703, issued January 6, 1987 disclose buspirone's use in treating panic disorders.
10. Alderdice discloses the use of buspirone in the improvement of short term memory in U.S. 4,687,772, issued August 18, 1987.
11. U.S. 4,777,173, issued October 10, 1988 to Shrotriya and Casten, discloses and claims the use of buspirone in treating alcohol abuse.

Buspirone and related members of the azapirone class of psychotropic agents share common pharmacological actions, due perhaps to similar interactions of these compounds with monoaminergic pathways in particular areas of brain. Specifically, functional binding of these agents at monoaminergic receptor sites in brain indicate a more or less common neuropharmacologic profile for azapirones that would render members of the class useful equivalents when applied to pharmacological applications involving neural mechanisms. The specific azapirones intended for use in the method of the present invention are shown in Table 1, infra.

The present invention resulted from an initial unexpected discovery that the representative azapirone, buspirone, was effective in treating patients suffering from sleep apneas. The only literature relating to the effects of any of the azapirones on respiration in general, concerned disclosures that buspirone had a stimulatory effect on respiration both in cats (Garner, et al., Am. Rev. Respir. Dis., 137:4, pages 946-950 (1989)) and in normal male volunteers (Rapoport, et al., Fed. Am. Soc. Exp. Biol., J 2:5, Abstract 7030 (1988).

Additionally, Rapoport, et al., Am. Rev. Respir. Dis., 139 (4), page A 625 (1989), suggested that buspirone may be a ventilatory stimulant in the unrestrained intact rat and may be effective in humans with hypoventilation syndroms.

It has become apparent that sleep apneas comprise a spectrum of related disorders with varying severity and morbidity. Sleep apneas have been usually classified as being an obstructive, central, or mixed apnea, depending on the presence or absence of respiratory efforts during the periods in which airflow has ceased. Obstructive and mixed apneas occur with greatest frequency with the most familiar being

obstructive sleep apnea syndrome in which sporadic recurring collapse of the patient's upper airway occurs during sleep. If the collapse is complete there is no air exchange at the nose and mouth and breathing is interrupted. The usual result is a partial arousal from sleep and a return to normal breathing. The patient in most instances has no knowledge or memory of these apnea episodes but finds himself constantly suffering from fatigue and daytime sleepiness for no apparent reason. These recurrent apnea episodes with resultant hypoxemia and fragmented sleep can have serious neurologic and cardiac consequences. In recent years there has been a growing awareness of the seriousness of the sleep apnea problem due to its wide occurrence and the lack of a recognized effective treatment.

Surgical and mechanical interventions as well as oxygen administration have been employed as treatments. None of these are very suitable. Treatment of sleep apneas by pharmacological intervention has also had little success. Respiratory stimulants, theophylline, antidepressants and progestational agents have been used to treat sleep apneas but have not been found to be very effective.

In sum, there is nothing in the prior art, including the specific references set forth hereinabove, that would suggest the use of buspirone or other azapirones for the treatment of sleep apneas.

## SUMMARY OF THE INVENTION

The present invention comprises the use of an azapirone or a pharmaceutically acceptable acid addition salt thereof for preparing a pharmaceutical composition for the treatment of patients suffering from sleep apneas. The pharmaceutical composition can be administered to a patient in need of such treatment. For oral administration a dose of from about 10 to 60 mg of said azapirone at the hour of sleep is usually employed.

## DETAILED DESCRIPTION OF THE INVENTION

The invention results from an initial discovery that buspirone administration is an effective treatment in preventing or reducing the incidence of sleep apneas. In the context of this invention, sleep apneas comprise all the sub-categories such as those caused by upper airway obstruction; those whose origins arise in the central nervous system; and those of a mixed type with contribution from both components. This invention is also intended for related respiratory disorders such as use in "sudden infant death syndrome" (SIDS).

It should be appreciated that no established therapy for sleep apneas exists. As an example, treatment modalities for the most common subtype of this class of disorders, chronic obstructive sleep apnea, have included weight loss, surgical removal of tissue that may cause obstruction, avoidance of all sedatives, mechanical interventions and oxygen masks. None of these measures has been effective in any consistent manner. In this as well as other sleep apneas, pharmacologic intervention has been used but effectiveness has not been established. Respiratory stimulants, antidepressants, theophylline and various progestational agents have been tried but not found to be very effective.

A recent review (Guilleminault, "Obstructive Sleep Apnea Syndrome", in Psychiatric Clinics of North America -Vol. 10. No. 4. pages 607-618 (1987)) reveals a widespread occurrence as well as a range of serious medical sequalae.

The method of the present invention then is intended to encompass the use of azapirone compounds of Formulas I and II. In Formula I,

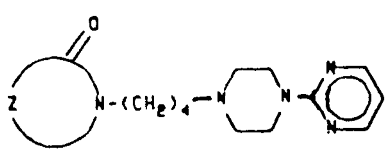

I

Z is a member selected from the group

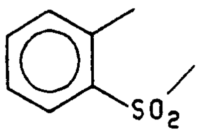 , 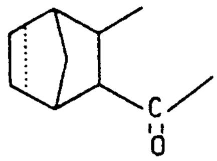 , 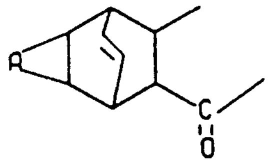 and 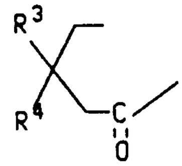

with $R^3$ and $R^4$ being independently selected from $C_{1-4}$ alkyl and hydrogen.
A can be $-CH_2-$, -O-, $-CH_2CH_2-$ or -CH=CH-. The dotted and solid line represents either a single or a double chemical bond.

The compound of Formula II differs somewhat in structure but possesses a similar pharmacologic profile.

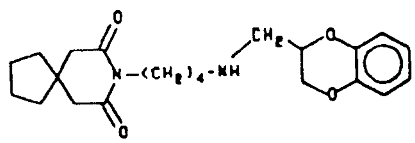

II

Preferred azapirones are the following compound, listed below in Table 1, which have been disclosed previously as psychotropic agents with useful anxiolytic properties.

Table 1
Specific Azapirone Compounds

| STRUCTURE | REFERENCE |
|---|---|
| 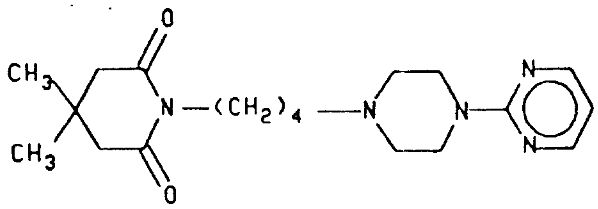 GEPIRONE | US 4,423,049 |
| 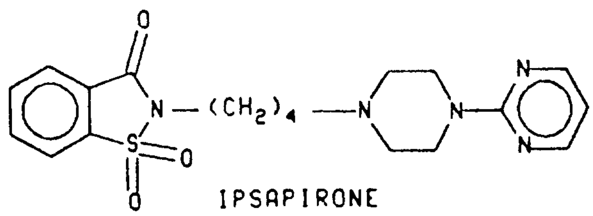 IPSAPIRONE | EP 129,128A |
| 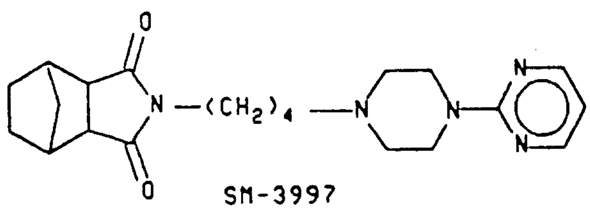 SM-3997 | US 4,507,303 |
| 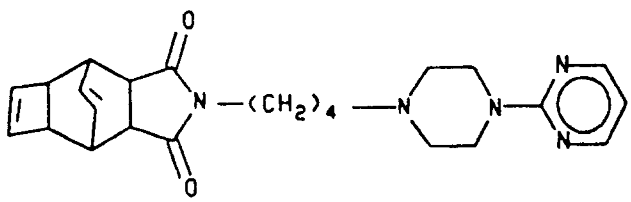 WY-47,846 | J.Med.Chem., 1988 1382-1392 |
| 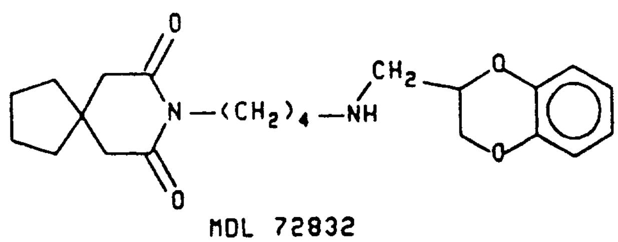 MDL 72832 | US 4,612,312 |

There are two aspects to the use of azapirones in treating sleep apneas. The first is that the administration of an azapirone effectively reduces the frequency and severity of the apnea episodes during sleep. This is reflected in significantly increased undisturbed sleep and a significant increase in blood oxygen levels. The second aspect involves azapirone alleviation of the symptomatology associated with the occurrence of sleep apneas. The azapirone treatment alleviates the sleep apnea-related symptoms of anxiety, depression, fatigue, malaise, irritability, anger and hostility.

Clinical experience with selected azapirones would indicate that maximum response levels to azapirone administration may require a period of chronic administration, in some instances on the order of two to four weeks. It is appreciated by those skilled in the art that the time to emergence of full therapeutic response, as well as the dosage level required, can vary from patient to patient.

The effectiveness of azapirone treatment of patients suffering from sleep apneas can be exemplified by clinical experience with buspirone. Single dose administration of buspirone, given at bedtime to patients suffering from obstructive sleep apnea, resulted in increased sleep efficiency with experimentally derived measurements showing a gain in total sleep time and a marked reduction in episodes of sleep disturbance. One of the most consistent physiological measurements of improvement was a 10 to 20% increase in blood oxygen levels, an indication of improved respiratory efficiency.

In summary, the present invention concerns the use of an azapirone for preparing a pharmaceutical composition for treating sleep apneas comprising obstructive, central and mixed apneas, in a patient population that ranges from infants to geriatric-aged individuals. The treatment involves administration of an azapirone given as the azapirone base or one of its pharmaceutically acceptable salts, in the form of a pharmaceutical composition, either alone or as an adjunct to their therapies.

Pharmaceutically acceptable acid addition salts of buspirone and methods of pharmaceutical formulation are described in the patents of Wu, et al., U.S. 3,717,634 and U.S. 3,976,776. Substitution of other azapirone agents may be made with slight variation in method or as set forth in the pertinent references given supra.

Administration of azapirone according to the present invention may be made by the parenteral, oral, or rectal routes. Parenteral administration comprises injection, e.g., intravenous or intramuscular injection, as well as any other parenteral route of administration. The oral route is preferred. The clinical dosage range for treatment of sleep apneas depend on age of recipient, body weight, general physical condition and severity of sleep apnea disorder. In general the drug treatment will be administered at the hour of sleep and will be about 20 to 60 mg for an average adult. Dosage will be appropriately reduced for infants and young children, according to the clinical judgement of the attending physician. Consistent with good clinical practice, dose adjustments may be made not only in consideration of the individual patient and the respiratory disorder but also in consideration of the specific azapirone selected for use in the method of the present invention. Such dosage adjustments are familiar to those skilled in clinical pharmacology and the medical arts.

## Claims

1. The use of an azapirone compound of formula I:

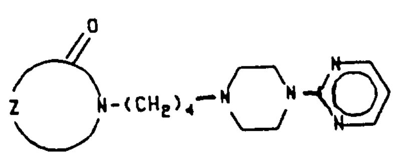

I

wherein

Z is a member selected from the group consisting of

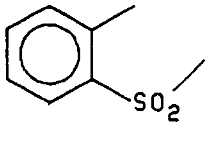 , 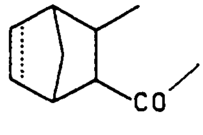 , 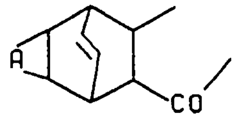 and 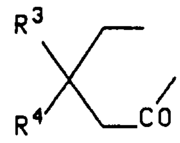

with the dotted and solid line representing either a single or a double chemical bond; A being selected, from the group consisting of O, $CH_2$, $CH_2CH_2$ and CH = CH; and

$R^3$ and $R^4$ being independently selected from hydrogen and $C_{1-4}$ alkyl,

or a pharmaceutically effective acid addition salt thereof for preparing a pharmaceutical composition for

treatment of sleep apneas.

2. The use of claim 1 wherein Z in the Formula I compound is

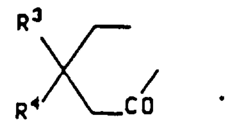

3. The use of claim 1 wherein Z in the Formula I compound is

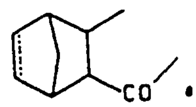

4. The use of claim 1 wherein Z in the Formula I compound is

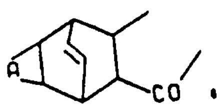

5. The use of claim 1 wherein Z in the Formula I is

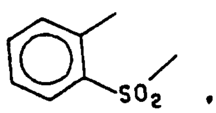

6. The use of claim 1 wherein the Formula I compound is gepirone.

7. The use of claim 1 wherein the Formula I compound is ipsapirone.

8. The use of claim 1 wherein the Formula I compound is SM 3997.

9. The use of claim 1 wherein the Formula I compound is WY 47846.

10. The use of an azapirone compound of formula II

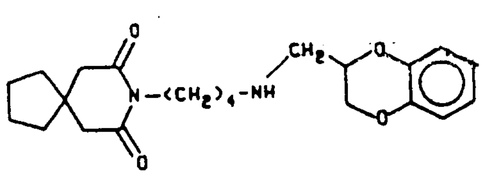

or a pharmaceutically effective acid addition salt thereof for preparing a pharmaceutical composition for treatment of sleep apnea.

11. The use of any one of the preceding claims wherein the azapirone compound is used as a hydrochloride salt.

## Patentansprüche

**1.** Verwendung einer Azapironverbindung der Formel I:

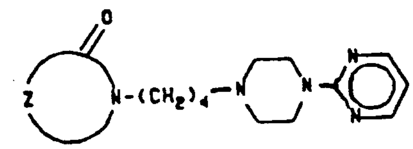

I

worin
Z ausgewählt ist unter

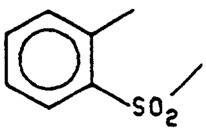 , 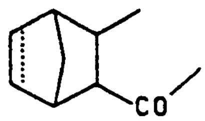 , 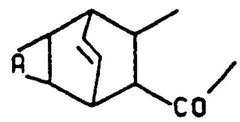 und 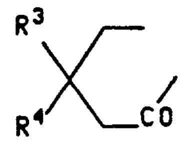

wobei die gepunktete und die durchgezogene Linie eine chemische Einfach- oder Doppelbindung darstellt; A ausgewählt ist unter O, $CH_2$, $CH_2CH_2$ und CH = CH; und
$R^3$ und $R^4$ unabhängig voneinander ausgewählt sind unter Wasserstoff und $C_{1-4}$-Alkyl,
oder eines pharmazeutisch wirksamen Säureadditionssalzes davon zur Herstellung einer pharmazeutischen Zusammensetzung zur Behandlung von Schlafapnoen.

**2.** Verwendung gemäß Anspruch 1, worin Z in der Verbindung der Formel I für

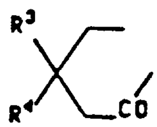

steht.

**3.** Verwendung gemäß Anspruch 1, worin Z in der Verbindung der Formel I für

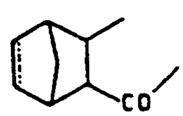

steht.

**4.** Verwendung gemäß Anspruch 1, worin Z in der Verbindung der Formel I für

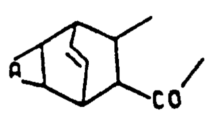

steht.

5. Verwendung gemäß Anspruch 1, worin Z in der Verbindung der Formel I für

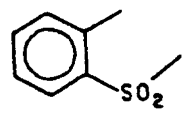

steht.

6. Verwendung gemäß Anspruch 1, wobei es sich bei der Verbindung der Formel I um Gepiron handelt.

7. Verwendung gemäß Anspruch 1, wobei es sich bei der Verbindung der Formel I um Ipsapiron handelt.

8. Verwendung gemäß Anspruch 1, wobei es sich bei der Verbindung der Formel I um SM 3997 handelt.

9. Verwendung gemäß Anspruch 1, wobei es sich bei der Verbindung der Formel I um WY 47846 handelt.

10. Verwendung einer Azapironverbindung der Formel II

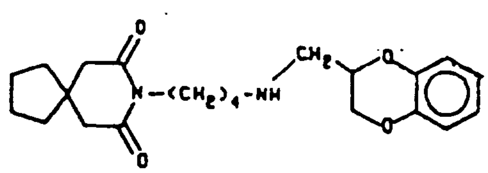

II

oder eines pharmazeutisch wirksamen Säureadditionssalzes davon zur Herstellung einer pharmazeutischen Zusammensetzung zur Behandlung von Schlafapnoe.

11. Verwendung gemäß einem der vorhergehenden Ansprüche, wobei die Azapironverbindung als Hydrochloridsalz verwendet wird.

## Revendications

1. Utilisation d'un composé de l'azapirone de formule I :

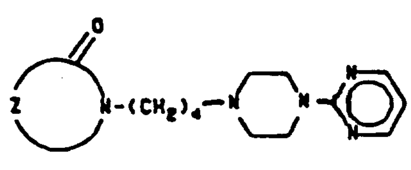

I

dans laquelle
Z est choisi parmi les groupes suivants

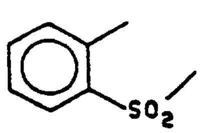 , 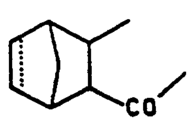 , 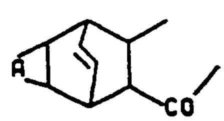 et 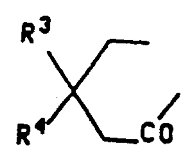

les lignes pleines et celles en pointillé représentent soit une liaison chimique simple soit une double; A

pouvant être O, $CH_2$,$CH_2CH_2$ ou CH = CH.
$R^3$ et $R^4$ étant indépendamment un hydrogène ou un alkyl en $C_1$-$C_4$.
ou un de leurs sels d'addition d'un acide efficace sur le plan pharmaceutique pour la préparation d'une composition pharmaceutique destinée au traitement des apnées du sommeil.

2. Utilisation selon la revendication 1 où Z, dans le composé de formule I, est

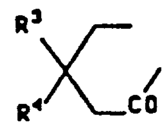

3. Utilisation selon la revendication 1 où Z, dans le composé de formule I, est

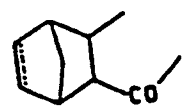

4. Utilisation selon la revendication 1 où Z, dans le composé de formule I, est

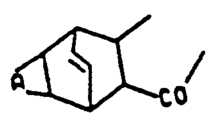

5. Utilisation selon la revendication 1 où Z, dans le composé de formule I, est

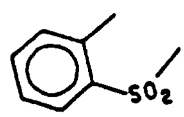

6. Utilisation selon la revendication 1 où le composé de formule I, est la gépirone.

7. Utilisation selon la revendication 1 où le composé de formule I, est l'ipsapirone.

8. Utilisation selon la revendication 1 où le composé de formule I, est le SM 3997.

9. Utilisation selon la revendication 1 où le composé de formule I, est le WY 47846.

10. Utilisation d'un composé d'azapirone de formule II

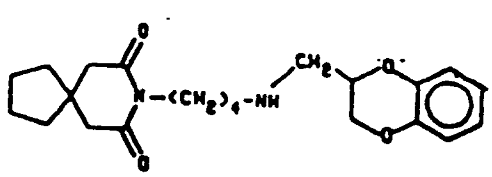

ou un de ses sels d'addition acide efficace sur le plan pharmaceutique, pour la préparation d'une

composition pharmaceutique destinée au traitement de l'apnée du sommeil.

**11.** Utilisation selon l'une des revendications précédentes, dans laquelle le composé d'azapirone est utilisé sous la forme d'un sel d'hydrochlorure.